(19)

Europäisches
Patentamt
European
Patent Office
Office européen
des brevets

(11) **EP 4 736 837 A1**

(12) **EUROPEAN PATENT APPLICATION**

(43) Date of publication:
 **06.05.2026   Bulletin 2026/19**

(21) Application number: **24209693.1**

(22) Date of filing: **30.10.2024**

(51) International Patent Classification (IPC):
 **A61K 8/66** *(2006.01)*    **A61Q 5/00** *(2006.01)*

(52) Cooperative Patent Classification (CPC):
 **A61K 8/66; A61Q 5/004**

(84) Designated Contracting States:
 **AL AT BE BG CH CY CZ DE DK EE ES FI FR GB GR HR HU IE IS IT LI LT LU LV MC ME MK MT NL NO PL PT RO RS SE SI SK SM TR**
 Designated Extension States:
 **BA**
 Designated Validation States:
 **GE KH MA MD TN**

(71) Applicant: **SkyLab AG**
 **1066 Epalinges (CH)**

(72) Inventors:
 • **Belous, Elena**
  **121309 MOSCOW (RU)**
 • **Ivanova, Angelina**
  **LONDON, NW61NE (GB)**

(74) Representative: **Glawe, Delfs, Moll Partnerschaft mbB Hopfenmarkt 33 20457 Hamburg (DE)**

(54) **LACCASE FOR REDUCING PIGMENT LEACHING FROM DYED HAIR**

(57)    The invention relates to a post-color hair care composition comprising laccase and having a pH of ≤ 7.0. Said composition protects dyed hair from fading or wash-out during exposure to air and/or shampooing.

Percentage of color loss after 14 times of washing

Fig. 1

**EP 4 736 837 A1**

**Description**

FIELD OF THE INVENTION

The present invention pertains to novel applications of Laccase for reducing pigment leaching from dyed hair, including its use for prolonging color vibrancy. The present invention can be applied in the field of hair care products.

BACKGROUND OF THE INVENTION

In recent years, there has been an increasing interest in self-image, which individuals project to others, influencing how they are perceived, particularly in the context of rising aspirations toward aesthetic appeal. Cosmetic products, including hair care products, play a critical role in this process. Hair is considered an important indicator of attractiveness and beauty, as well as a means of expressing gender identity.

Regardless of gender, age, income level, or social status, individuals resort to chemical hair treatments, highlighting the importance of appearance in modern society. Modern technologies allow for easy alteration of hairstyles through bleaching, dyeing, and perming, using a variety of natural and synthetic components. This trend contributes to the growing global interest in chemical hair transformation products.

Statistical data indicates that in the United States and Europe, between 50% and 80% of women, and approximately 10% of men over 40, have experienced hair dyeing. Analysis of trends in hair dye use shows the stability of these figures over the past decades [1].

Hair dyeing involves treating hair with various chemical compounds to change its color. Based on the duration of the color's presence on the hair, these products are classified as either temporary or permanent. This classification depends on the type of active substances used in the dyeing process, and the methods, which are divided into non-oxidative and oxidative hair coloring agents.

Permanent hair dyes typically contain active chemical components that do not color the hair directly but undergo oxidation to achieve the desired shade, leading to the term "oxidative hair dye". The main ingredients in oxidative dyes are a precursor and an oxidizing agent. Quinonediimine intermediates are transient products formed upon interaction with hydrogen peroxide, which serves as the developer. The resulting interaction between the oxidizing agent and these intermediates forms the dye molecule. It is important to note that successful dyeing requires both an alkaline environment and an oxidizer-most commonly hydrogen peroxide-allowing the colorants to penetrate deeply into the hair cuticle [2].

Hair dyeing is becoming increasingly popular; however, the rapid fading of the newly applied color poses a significant challenge for consumers. This phenomenon is primarily related to aesthetic concerns: hair loses its brightness and color intensity within just a few weeks after the procedure, even before noticeable regrowth.

There are two main approaches to addressing this issue. The first is to develop more long-lasting dyes, and the second is to slow down pigment washout. The primary loss of color in dyed hair usually occurs due to dye leaching during regular shampooing and exposure to external factors such as ultraviolet radiation, which can degrade dye molecules. However, studies show that shampooing is the most significant factor in color loss, while UV exposure becomes notable only after 90 hours of intense exposure.

The mechanism behind hair color fading caused by shampooing is not yet fully understood. Nevertheless, several key factors influencing this process have been identified. One of these is the solubility of the dye in water. Most dyes used for hair coloring are water-soluble, and the rate at which they are removed by shampooing depends on this characteristic. The solubility of the dye is determined by its chemical structure, and typically bi-nuclear dyes exhibit higher solubility. Red dyes, in particular, show more pronounced fading, as they are more water-soluble and thus more prone to washout.

Another factor is the extent of hair damage. Damaged hair has a more porous structure, making it easier for dye molecules to be released during washing. As a result, bleached hair loses color more quickly than unbleached hair, as it loses the protective F-layer and increases pore size within cuticle cells. Additionally, when exposed to water, the cuticle and cortex swell, creating additional pathways for dye leaching. Surfactants (SLS) contained in shampoos and conditioners contribute to the wetting and saturation of the hair shaft, further facilitating the leaching of dye molecules.

Consumers seek to maintain color vibrancy until the next oxidative process, which implies protecting dyed hair from fading for up to six weeks - the period required for color renewal at the noticeable regrowth stage. Meeting this demand remains a pressing challenge for the cosmetic industry. Color protection is considered a crucial focus in the hair care product market. In recent years, there has been a significant expansion in the range of color-protective products, most of which are presented as shampoos and conditioners. Some of these products contain silicones that form a hydrophobic film on the hair surface, which, according to several studies, helps preserve color [3].

Tests have shown that mixtures of dimethiconol/dimethicone can reduce pigment washout from hair, while silicone resins such as trimethylsiloxysilicate or propylphenylsilsesquioxane are effective in minimizing color fading caused by UV exposure [4]. However, frequent use of hair products containing dimethicone may lead to to dry and brittle hair [Madnani N, Khan K. Hair cosmetics. Indian J Dermatol Venereol Leprol. 2013 Sep-Oct;79(5):654-67. doi: 10.4103/0378-6323.116734. PMID: 23974582.]. Dimethicone, like dimethiconol, weighs down hair at high concentrations, potentially giving it a greasy appearance.

In an article discussing the durability of Level 2 (also known as semi-permanent) and Level 3 (oxidative or permanent) hair dyes, an olefinic conditioner is described, formulated for the grafting of ethylene/acrylate and acrylamidomethacrylate copolymers. When used in concentrations of 2-5%, this conditioner enhances color retention by 15% for Level 2 dyes and 5% for Level 3 dyes (compared to untreated hair) when incorporated into two-component dyes, rather than being applied as a post-dyeing conditioner. The proposed mechanism for this function involves the formation of an olefinic occlusive barrier on the surface of the hair [5]. However, the olefinic conditioner may cause scalp irritation and impart a drying effect.

U.S. Patents 7066966 and 7147672 describe an oxidative dye composition for keratin fibers containing cationic poly(vinyl lactam) [6,7].

Hydrophobically modified cationic polymers, as well as their combinations with quaternizing agents, demonstrate high efficacy in protecting colored hair from dye washout caused by regular shampooing. These hydrophobically modified cationic polymers possess both hydrophobic segments and sufficient cationic charge density, which provides structural strength to the hair while simultaneously creating a hydrophobic effect. This contributes to the "locking" of the dye within the hair, even with daily washing [8]. However, there is a growing consumer preference for natural ingredients in hair care formulations, calling into question the use of synthetic polymer components.

Laccases are multi-component copper-containing oxidases that catalyze the oxidation of a wide variety of compounds, accompanied by the four-electron reduction of molecular oxygen to water ($H_2O$). These enzymes can oxidize both phenolic and non-phenolic substrates. Laccases are derived from various sources, including plants, actinomycetes, fungi (ascomycetes, basidiomycetes, and deuteromycetes), and bacteria. Due to their versatility in oxidizing a broad range of substrates, laccases find applications in numerous biotechnological processes, such as biological bleaching of cellulose, food industry processes, textile dye degradation, and bioremediation of xenobiotics.

One of the less explored applications of laccase is in the cosmetic industry, specifically in hair dyeing. Currently, the market is dominated by synthetic hair dyes; however, their use is associated with various side effects and damage to the hair structure. In addition to oxidative reactions, laccases are known to catalyze the polymerization of certain substrates, which opens possibilities for synthesizing dyes based on natural precursors. The article [9] discusses the potential of laccases for dye synthesis, as these enzymes effectively catalyze the polymerization of phenolic compounds, the products of which can be utilized in the development of safe and effective hair dyes.

In study [10], the potential for using laccase to develop hair dyes in three color variants-black, brown, and red-was evaluated. The effect of adding this enzyme on color fastness during shampoo washing was assessed, with experiments focusing exclusively on a limited set of phenolic compounds: gallic acid, syringic acid, ferulic acid, catechin, and catechol. It is important to note that the results of this study do not provide conclusions about laccase's effect on the color fastness of other shades (beyond black, brown, and red), nor on dyes containing untested polyphenols. Furthermore, the evaluation of laccase potential was conducted solely within the context of its use in hair dye manufacturing.

Laccase, in combination with the aforementioned phenolic compounds, was investigated for its effect on pigment retention against washout [11]. Hair pigment was washed using shampoo, and the results demonstrated a positive impact of incorporating laccase into the dye formulation with polyphenols to enhance color retention. However, in this study, laccase was only included as part of the dye formulation and was examined in combination with polyphenols.

Additionally, laccase was studied in [12], where laccase-mediated oxidation of dihydroxyphenylalanine was utilized in the dyeing process. The authors claim that the method of laccase-mediated polymerization of dihydroxyphenylalanine and simultaneous dyeing can be used for dyeing hair black. This study is limited to assessing the impact of the active on only one color, with laccase being used exclusively as part of the dye formulation.

The authors hypothesized that the application of laccase in shampoo and conditioner formulations could help reduce the rate of pigment washout from dyed hair and prolong color vibrancy. However, this assumption is limited by the lack of research on laccase's effect on reducing pigment washout when incorporated into shampoos and conditioners rather than dyes. Moreover, most studies on dye formulations containing laccase also included polyphenolic compounds (from a limited list), which suggests a synergy between the investigated polyphenols and laccase. The absence of such synergy may diminish the effectiveness of laccase when used without these polyphenols in shampoo and conditioner formulations, as opposed to dyes. This is a crucial factor since hair dyes typically have an alkaline pH, whereas shampoos and conditioners are acidic. In earlier studies, laccase was considered an active component only in alkaline environments, providing no basis to assume that its activity would be preserved with a change in pH. Additionally, the tests on increased color fastness were conducted for a limited range of colors (black, brown, and red), which are typically more prone to rapid washout. Thus, there is no reason to assume that laccase would have a similar effect on reducing pigment washout for other shades.

INVESTIGATIONS

**Example 1.** In-vitro investigation of color fastness when shampooing hair with and without laccase

1.1 Materials and methods

1.1.1 Investigated compound

Table 1. Investigated compound

| No. | Ingredient | CAS |
|-----|-----------|-----|
| 1 | Laccase | 80498-15-3 |

1.1.2. Hair Samples

Light colored bleached hair samples of 48 pieces of bleached hair were used in the study. The strands had not been previously colored or additionally treated in any way.

1.1.3 Coloring

The hair samples were dyed using commercial hair dyes in accordance with the manufacturer's instructions. Five distinct colors were applied: blue, emerald, red, copper, and violet. The specific number of strands dyed in each color is detailed in Table 2. The dye preparation involved a tint and an oxidant, with the ratio set at 1:1.5, as recommended by the manufacturer. This ratio was selected due to the strands being previously bleached to a light shade and dyed for the first time. The freshly mixed dye was applied to the strands, which were then wrapped in aluminum foil to simulate a natural dyeing process. The strands remained in the foil for 30 minutes, adhering to the guidelines provided by the dye manufacturer.

Table 2. Number of strands dyed in a particular color

| Color | Samples Quantity |
|-------|-----------------|
| Blue | 6 |
| Emerald | 6 |
| Red | 12 |
| Copper | 12 |
| Violet | 12 |

1.1.4 Hair washing with shampoo

Following this initial dye application, the strands were removed from the foil, rinsed under running water, and subjected to two washes with commercially procured shampoo. Four identical shampoos were utilized: Shampoo No. 1 remained unaltered, while Shampoo No.2 was supplemented with laccase at a concentration of 0.5%, Shampoo No.3 was supplemented with laccase at a concentration of 0.25%, Shampoo No.4 was supplemented with laccase at a concentration of 0.05%. A total of 24 strands were rinsed with Shampoo No.1, 8 strands with Shampoo No.2, 8 strands with Shampoo No.3, 8 strands with Shampoo No.4. The distribution of strand numbers, colorants, and rinsing shampoos is presented in Table 3.

After the initial rinse, the strands were air-dried at room temperature, and their color was measured using a colorimeter based on the parameter L. Subsequently, all strands underwent 14 washes with Shampoo No.1, Shampoo No.2, Shampoo No.3 and Shampoo No.4 according to the distribution outlined in Table 3, simulating a washing regimen over six weeks post-coloring. Following this washing regimen, hair color was re-evaluated using the colorimeter, as detailed in section 1.1.5.

Table 3. Distribution of samples by staining with specific colors and washing with specific shampoos

| Sample No. | Color | Shampoo No. |
|---|---|---|
| 1 | Blue | 1 |
| 2 | Blue | 2 |
| 3 | Blue | 1 |
| 4 | Blue | 3 |
| 5 | Blue | 1 |
| 6 | Blue | 4 |
| 7 | Emerald | 1 |
| 8 | Emerald | 2 |
| 9 | Emerald | 1 |
| 10 | Emerald | 3 |
| 11 | Emerald | 1 |
| 12 | Emerald | 4 |
| 13 | Red | 1 |
| 14 | Red | 2 |
| 15 | Red | 1 |
| 16 | Red | 2 |
| 17 | Red | 1 |
| 18 | Red | 3 |
| 19 | Red | 1 |
| 20 | Red | 3 |
| 21 | Red | 1 |
| 22 | Red | 4 |
| 23 | Red | 1 |
| 24 | Red | 4 |
| 25 | Copper | 1 |
| 26 | Copper | 2 |
| 27 | Copper | 1 |
| 28 | Copper | 2 |
| 29 | Copper | 1 |
| 30 | Copper | 3 |
| 31 | Copper | 1 |
| 32 | Copper | 3 |
| 33 | Copper | 1 |
| 34 | Copper | 4 |
| 35 | Copper | 1 |
| 36 | Copper | 4 |
| 37 | Violet | 1 |
| 38 | Violet | 2 |
| 39 | Violet | 1 |

(continued)

| Sample No. | Color | Shampoo No. |
|---|---|---|
| 40 | Violet | 2 |
| 41 | Violet | 1 |
| 42 | Violet | 3 |
| 43 | Violet | 1 |
| 44 | Violet | 3 |
| 45 | Violet | 1 |
| 46 | Violet | 4 |
| 47 | Violet | 1 |
| 48 | Violet | 4 |

1.1.5 Color analysis method

Color changes were measured by using colorimeter. Color readings of L parameter were obtained with "specular included" and D65 light source settings. Hair color changes after coloring and the first washing were determined by dE. dE was calculated using the next formulas to evaluate color change:

$$dE = L_t - L_0$$

where $L_0$ and $L_t$ are measured L color parameter before and after the wash cycles, respectively. The larger value of dE reflects a greater change of color. The values of L readings on each hair tress represent an average of three measurements, each corresponding to a different site on the hair but within the same area before and after washes. The amount of color protection from a treatment is calculated:

$$\% \, Color \, protection \, = \, \% \, dE \, = \frac{(dE_1 - dE_n) \times 100}{dE_1}$$

where $dE_1$ - indicator for samples washed with shampoo No. 1 and $dE_n$ with shampoo No.2, 3 or 4.

1.3. Results

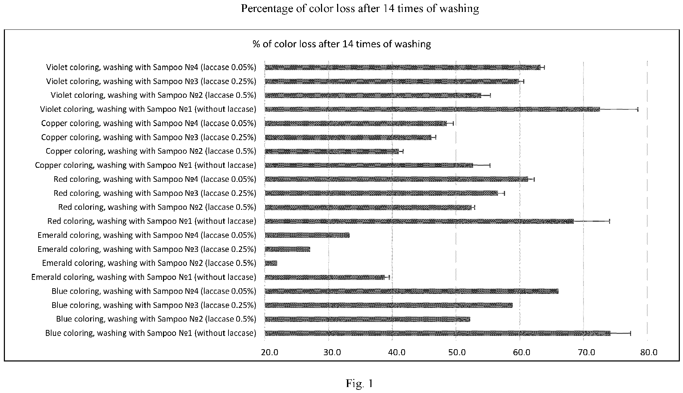

Table 4 describes the results of color evaluation on the colorimeter after staining, after intensive rinsing, percentage of color loss. Figure 1 illustrates the results of color leaching from hair samples, categorized by hue. This categorization is essential as the leaching rates of different colors vary significantly.

The values presented in Figure 1 are calculated as follows: for the colors Blue and Emerald, the data for strands washed with shampoo No. 1 are derived from the arithmetic mean of three measurements taken for each of the three strands of each color. The data for strands of the same colors that were washed with shampoos No. 2, 3, and 4 are also calculated as the arithmetic mean of three measurements for each strand of each color. For the colors Red, Copper, and Violet, the data for strands washed with shampoo No. 1 are obtained by taking the arithmetic mean of three measurements from each of the six strands of each color. The data for strands of the same colors washed with shampoos No. 2, 3, and 4 are derived from the arithmetic mean of three measurements taken from two strands of each color.

The data indicate that purple, blue, and red exhibited greater washout compared to copper and Emerald. Consequently, a comparative analysis was conducted based on color. Notably, across all five tested colors, the incorporation of laccase in the shampoo formulation was found to mitigate color washout. In the color washout investigation, a total of 14 cycles were executed to simulate the frequency of scalp washing over a six-week duration. This experimental design substantiates the conclusion that the incorporation of laccase into both shampoo and conditioner formulations effectively reduces the extent of pigment washout. This reduction persists until new hair growth occurs, at which point reapplication of the color treatment will be necessary. Laccase demonstrated effective activity within a concentration range of 0.05% to 0.5%, with elevated concentrations further enhancing the reduction of color leaching. Additionally, laccase exhibited both activity and stability in formulations with a pH range of 4.5 to 7 typical for shampoos and conditioners, indicative of an acidic environment, whereas previous studies primarily focused on its performance in alkaline conditions. The enzyme was effective against all dyes assessed, confirming its capability to diminish the leachability of various colors from dyed hair to differing extents, attributable to the inherent differences in leaching tendencies among the colors.

REFERENCES

1. He Y, Cao Y, Nie B, Wang J. Mechanisms of impairment in hair and scalp induced by hair dyeing and perming and potential interventions. Front Med (Lausanne). 2023 May 18;10:1139607. doi: 10.3389/fmed.2023.1139607. PMID: 37275367; PMCID: PMC10232955.
2. Yildmm A. et al. The chemistry mechanism of hair dyes //Middle East Journal of Science. - 2022. - T. 8. - No.2. - C. 173-193.
3. Zhou Y. et al. Protection of oxidative hair color fading from shampoo washing by hydrophobically modified cationic polymers //Journal of cosmetic science. - 2009. - T. 60. - No.2. - C. 217-238.
4. Schlosser A. Silicones used in permanent and semi-permanent hair dyes to reduce the fading and color change process of dyed hair occurred by wash-out or UV radiation. J Cosmet Sci. 2004;55 Suppl:S123-31. PMID: 15645088.
5. Martin, T , and Burns, T., "Novel Graft Polymers Boost Hair Color Retention", HAPPI, pp 92-95, October 2004
6. US patent No.7066966 «Oxidation dyeing composition for keratin fibers comprising a cationic poly(vinyllactam)» URL: https://patents.justia.com/patent/7066966
7. US patent No.7147672 « Oxidation dyeing composition for keratin fibers comprising a cationic poly(vinyllactam) and at least one C10-C14 fatty acid, methods and devices for oxidation dyeing» URL: https://patents.justia.com/patent/7147672
8. Patent No.WO2009085838A1 «Method of protecting dyed hair color from fading or wash-out» URL: https://patents.google.com/patent/WO2009085838A1/en
9. Kumar D, Kumar A, Sondhi S, Sharma P, Gupta N. An alkaline bacterial laccase for polymerization of natural precursors for hair dye synthesis. 3 Biotech. 2018 Mar;8(3):182. doi: 10.1007/s13205-018-1181-7. Epub 2018 Mar 14. PMID: 29556436; PMCID: PMC5851942.
10. Jeon JR, Kim EJ, Murugesan K, Park HK, Kim YM, Kwon JH, Kim WG, Lee JY, Chang YS. Laccase-catalysed polymeric dye synthesis from plant-derived phenols for potential application in hair dyeing: Enzymatic colourations driven by homo- or hetero-polymer synthesis. Microb Biotechnol. 2010 May;3(3):324-35. doi: 10.1111/j.1751-7915.2009.00153.x. Epub 2009 Nov 11. PMID: 21255331; PMCID: PMC3815374.
11. Panwar V, Dey B, Sheikh JN, Dutta T. Thermostable bacterial laccase for sustainable dyeing using plant phenols. RSC Adv. 2022 Jun 21;12(28):18168-18180. doi: 10.1039/d2ra02137d. PMID: 35800313; PMCID: PMC9210865.
12. Jia W. et al. Laccase-mediated in situ oxidation of dopamine for dyeing of human hair //Fibers and Polymers. - 2021. - T. 22. - C. 141-148.

**Claims**

1. A post-color hair care composition comprising laccase and having a pH of $\leq$ 7.0.

2. The composition of claim 1, wherein said composition has a pH in the range of 4.5 - 7.0.

3. The composition of claim 1 or claim 2, wherein said laccase is present in said composition in an amount of at least 0.05% by weight of said composition, preferably at least 0.25% by weight, further preferably at least 0.5% by weight or in an amount of a range selected from the following: 0.01-10% by weight, 0.01-5% by weight, 0.01-3.0% by weight, 0.01-1.0% by weight, 0.05-10% by weight, 0.05-5% by weight, 0.05-3.0% by weight, 0.05-1.0% by weight, 0.1-10% by weight, 0.1-5% by weight, 0.1-3.0% by weight, 0.1-1.0% by weight, 0.25-10% by weight, 0.25-5% by weight, 0.25-3.0% by weight, 0.25-1.0% by weight, 0.5-10% by weight, 0.5-5% by weight, 0.5-3.0% by weight, 0.5-1.0% by weight.

4. The composition of any of the preceding claims, said composition not comprising a phenolic compound and/or a hair coloring dye.

5. The composition of claim 4, wherein said phenolic compound is selected from the group consisting of gallic acid, syringic acid, ferulic acid, catechin and catechol.

6. The composition of any of the preceding claims, wherein said hair dye color is selected from the following: blue, emerald, red, copper and violet.

7. The composition of any of the preceding claims, wherein said composition is formulated for application on a subject's hair.

8. The composition of any of the preceding claims, wherein said composition is a formulation selected from the following: liquid, aerosol, suspension, solution, tincture, cream, paste, lotion, ointment, gel, balsam, foam, shampoo and conditioner.

9. The composition of any of the preceding claims, wherein said composition is for protecting dyed hair color from fading or wash-out during exposure to air and/or shampooing, preferably for protecting said dyed hair of a living subject, wherein said living subject is further preferably a human or an animal.

10. Use of the composition of any of the preceding claims in the non-medical post-color treatment of dyed hair of a subject, including cosmetic and aesthetic treatment.

11. The use of claim 10, wherein said non-medical treatment protects said dyed hair from fading or wash-out during exposure to air and/or shampooing, wherein preferably said treatment protects said dyed hair of a living subject, wherein said living subject is further preferably a human or an animal.

12. The use of laccase in the manufacture of composition of any of claims 1-9.

13. A post-color treatment method of protecting dyed hair color from fading or wash-out during exposure to air and/or shampooing, said method comprising treating the dyed hair with a composition of any of claims 1-9.

14. The method of claim 13, wherein said treatment comprises treating said dyed hair in a leave-in manner with said composition.

15. The method of claim 13 or claim 14, wherein said method protects said dyed hair from said fading or wash-out for up to six weeks.

Percentage of color loss after 14 times of washing

Fig. 1

Color analysis results: percentage of color loss for samples and their visualization

| Sample No. | % of color loss | Hair samples after coloring | Hair samples after wash cycles |
|---|---|---|---|
| 1 | -66,91 | | |
| 2 | -52,17 | | |
| 3 | -77,74 | | |
| 4 | -58,82 | | |

| | | | |
|---|---|---|---|
| 5 | -78,05 | | |
| 6 | -66,03 | | |
| 7 | -37,25 | | |
| 8 | -21,88 | | |
| 9 | -40,14 | | |

| | | | |
|---|---|---|---|
| 10 | -27,02 | | |
| 11 | -39,07 | | |
| 12 | -33,24 | | |
| 13 | -47,77 | | |
| 14 | -40,15 | | |

| | | | |
|---|---|---|---|
| 15 | -60,79 | | |
| 16 | -41,98 | | |
| 17 | -56,56 | | |
| 18 | -45,19 | | |
| 19 | -49,91 | | |

| 20 | -47,11 | | |
| 21 | -47,14 | | |
| 22 | -47,11 | | |
| 23 | -53,86 | | |
| 24 | -50,01 | | |

| 25 | -80,20 | | |
|----|--------|---|---|
| 26 | -51,76 | | |
| 27 | -56,72 | | |
| 28 | -53,14 | | |
| 29 | -63,51 | | |
| 30 | -55,22 | | |

| 31 | -55,64 | | |
| 32 | -58,02 | | |
| 33 | -79,37 | | |
| 34 | -62,65 | | |
| 35 | -75,46 | | |
| 36 | -60,01 | | |

| 37 | -81,93 | | |
|----|--------|---|---|
| 38 | -51,89 | | |
| 39 | -74,30 | | |
| 40 | -56,02 | | |
| 41 | -85,81 | | |
| 42 | -60,94 | | |

| 43 | -59,89 | | |
|----|--------|---|---|
| 44 | -58,73 | | |
| 45 | -71,33 | | |
| 46 | -62,45 | | |

| | | | |
|---|---|---|---|
| 47 | -61,15 | | |
| 48 | -64,13 | | |

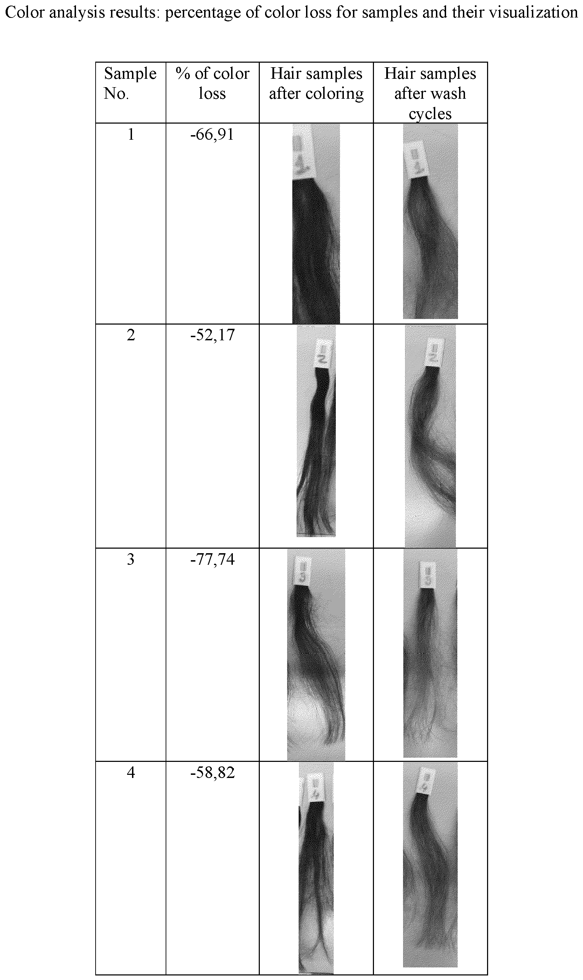

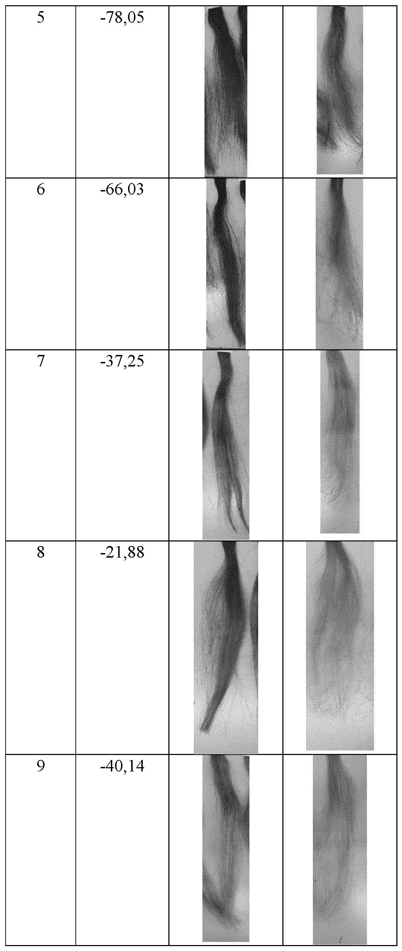

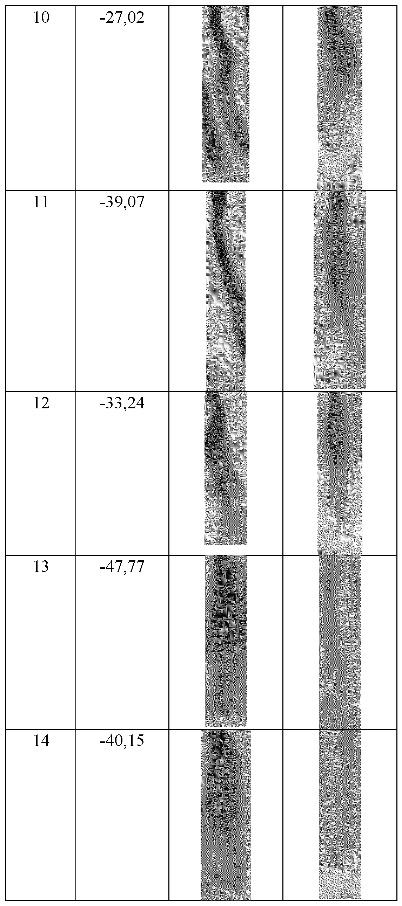

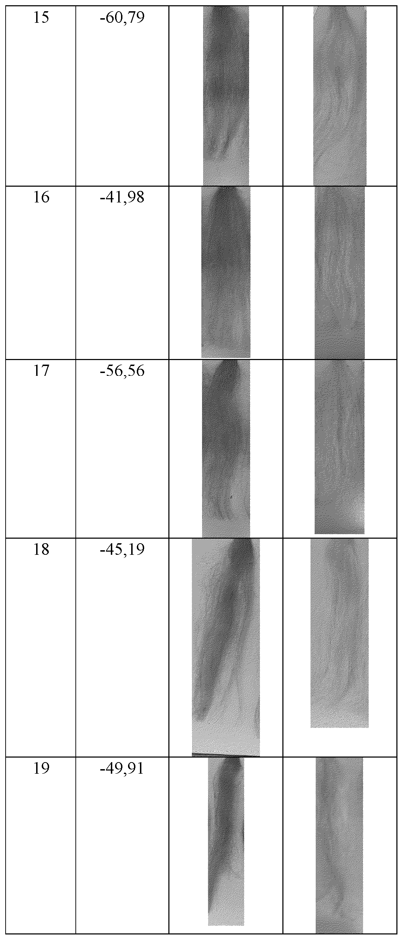

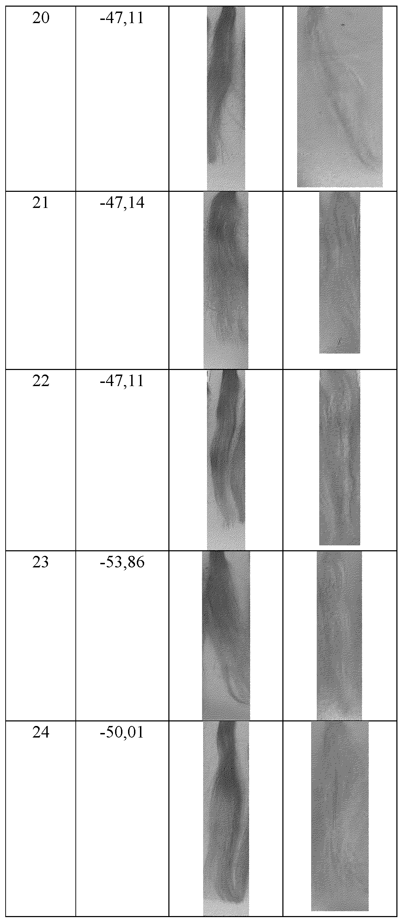

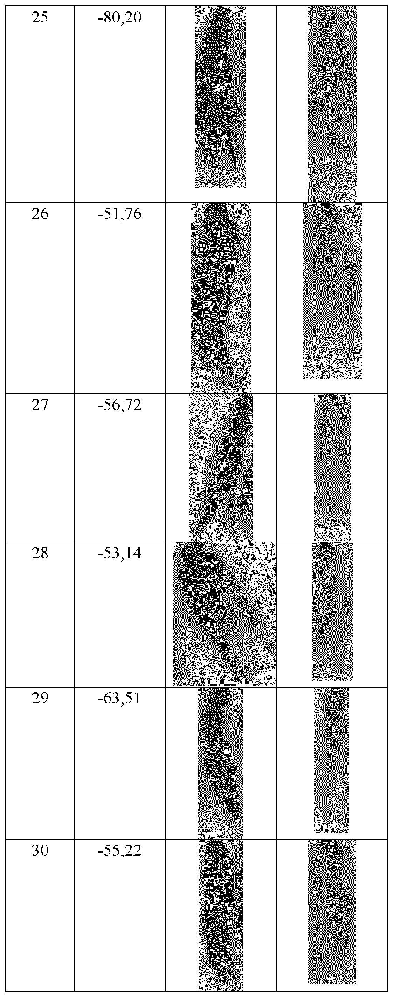

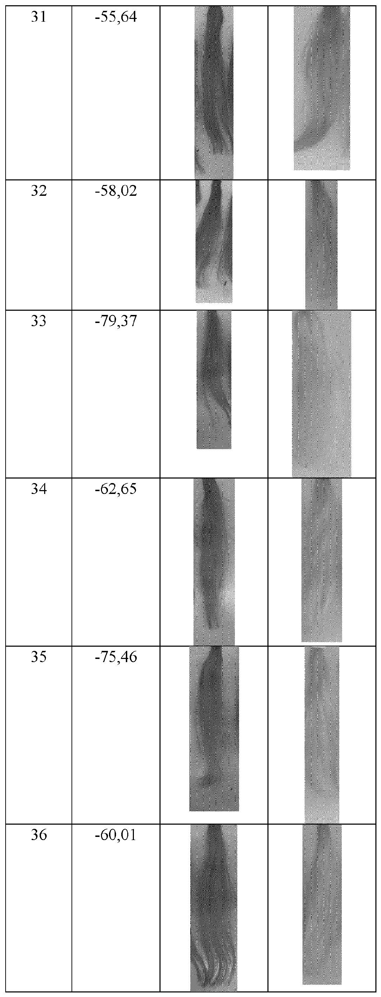

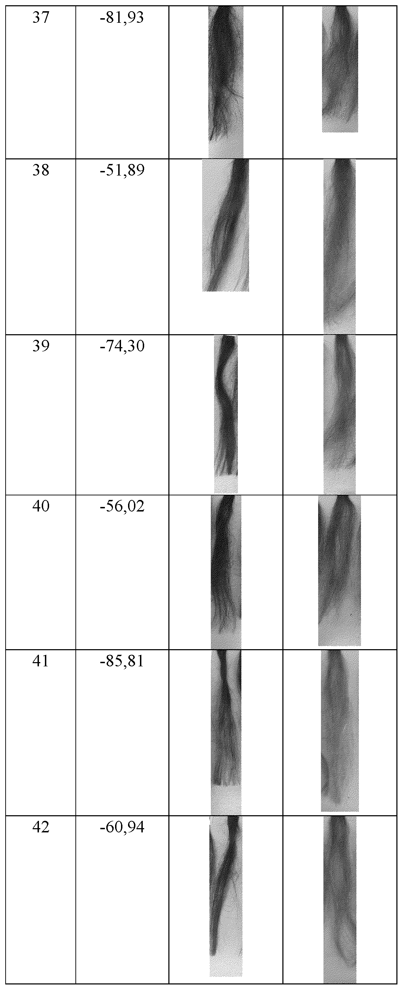

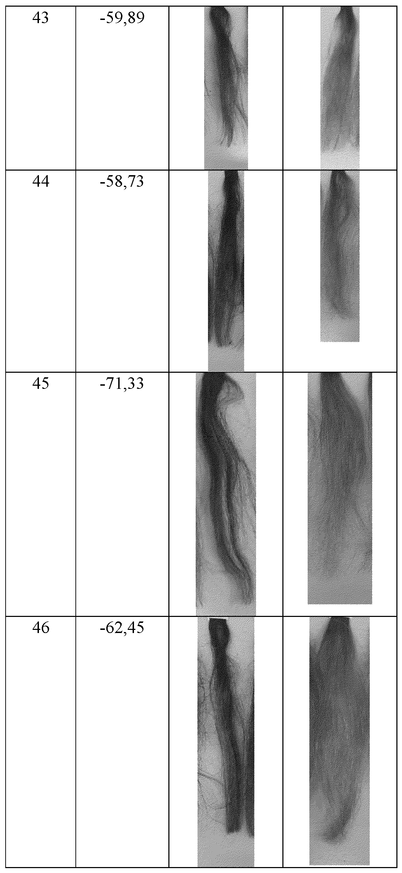

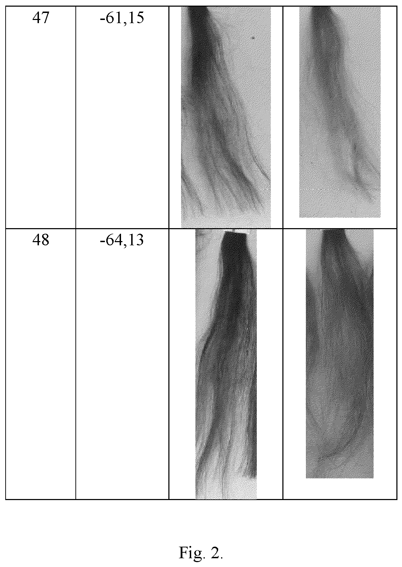

Fig. 2.

Europäisches
Patentamt

European
Patent Office

Office européen
des brevets

## EUROPEAN SEARCH REPORT

**Application Number**

EP 24 20 9693

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/261198 A1 (KAINZ SABINE [DE] ET AL) 30 December 2004 (2004-12-30) | 1-3, 6-13,15 | INV. A61K8/66 |
| Y | * claims 1,8,16,17,23 * <br> * paragraphs [0051], [0059], [0111] * <br> * example * * <br> * paragraphs [0232] - [0252]; example 7 * | 1-15 | A61Q5/00 |
| | ----- | | |
| X | EP 4 385 491 A1 (SKYLAB AG [CH]) 19 June 2024 (2024-06-19) <br> * claim 12 * <br> * paragraph [0001] * <br> * example 7 * | 1-9,12 | |
| | ----- | | |
| X | US 2014/335038 A1 (BHOGAL RANJIT KAUR [GB] ET AL) 13 November 2014 (2014-11-13) | 1,2, 6-10,12, 15 | |
| Y | * paragraphs [0001], [0017] - [0021]; claim 1; examples 4-6 * | 1-15 | |
| | ----- | | |
| X | EP 1 342 466 A1 (OREAL [FR]) 10 September 2003 (2003-09-10) | 1-3,6-9, 12 | TECHNICAL FIELDS SEARCHED (IPC) |
| Y | * paragraph [0047]; claims 1,7,17,18; example 1 * | 1-15 | A61K |
| | ----- | | A61Q |
| Y | WO 2009/085838 A1 (ISP INVESTMENTS INC [US]; YAN ZHOU [US] ET AL.) 9 July 2009 (2009-07-09) <br> * claims 1,15 * | 14 | |
| | ----- | | |

The present search report has been drawn up for all claims

| Place of search | Date of completion of the search | Examiner |
|---|---|---|
| Munich | 24 March 2025 | Grenouillat, N |

EPO FORM 1503 03.82 (P4C01)

# EP 4 736 837 A1

## ANNEX TO THE EUROPEAN SEARCH REPORT
## ON EUROPEAN PATENT APPLICATION NO.

EP 24 20 9693

This annex lists the patent family members relating to the patent documents cited in the above-mentioned European search report.
The members are as contained in the European Patent Office EDP file on
The European Patent Office is in no way liable for these particulars which are merely given for the purpose of information.

24-03-2025

| Patent document cited in search report | | Publication date | Patent family member(s) | | Publication date |
|---|---|---|---|---|---|
| US 2004261198 | A1 | 30-12-2004 | AU | 2002360987 A1 | 09-07-2003 |
| | | | DE | 10163052 A1 | 17-07-2003 |
| | | | EP | 1455736 A1 | 15-09-2004 |
| | | | US | 2004261198 A1 | 30-12-2004 |
| | | | WO | 03053375 A1 | 03-07-2003 |
| EP 4385491 | A1 | 19-06-2024 | EP | 4385491 A1 | 19-06-2024 |
| | | | WO | 2024126636 A1 | 20-06-2024 |
| US 2014335038 | A1 | 13-11-2014 | BR | 112014014242 A2 | 13-06-2017 |
| | | | CN | 103987369 A | 13-08-2014 |
| | | | EP | 2790658 A2 | 22-10-2014 |
| | | | US | 2014335038 A1 | 13-11-2014 |
| | | | WO | 2013087644 A2 | 20-06-2013 |
| EP 1342466 | A1 | 10-09-2003 | AT | E375784 T1 | 15-11-2007 |
| | | | BR | 0300550 A | 10-08-2004 |
| | | | CA | 2421092 A1 | 01-09-2003 |
| | | | DE | 60316860 T2 | 31-07-2008 |
| | | | EP | 1342466 A1 | 10-09-2003 |
| | | | FR | 2836632 A1 | 05-09-2003 |
| | | | JP | 2003286140 A | 07-10-2003 |
| WO 2009085838 | A1 | 09-07-2009 | BR | PI0821464 A2 | 16-06-2015 |
| | | | EP | 2234673 A1 | 06-10-2010 |
| | | | US | 2011219552 A1 | 15-09-2011 |
| | | | WO | 2009085838 A1 | 09-07-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7066966 B
- US 7147672 B

- WO 2009085838 A1


**Non-patent literature cited in the description**

- **MADNANI N** ; **KHAN K.** *Hair cosmetics. Indian J Dermatol Venereol Leprol.*, September 2013, vol. 79 (5), 654-67
- **HE Y** ; **CAO Y** ; **NIE B** ; **WANG J.** Mechanisms of impairment in hair and scalp induced by hair dyeing and perming and potential interventions.. *Front Med (Lausanne).*, 18 May 2023, vol. 10, 1139607
- **YILDMM A. et al.** The chemistry mechanism of hair dyes. *Middle East Journal of Science*, 2022, vol. 8 (2), 173-193
- **ZHOU Y. et al.** Protection of oxidative hair color fading from shampoo washing by hydrophobically modified cationic polymers. *Journal of cosmetic science*, 2009, vol. 60 (2), 217-238
- **SCHLOSSER A.** Silicones used in permanent and semi-permanent hair dyes to reduce the fading and color change process of dyed hair occurred by wash-out or UV radiation. *J Cosmet Sci.*, 2004, vol. 55, S123-31

- **MARTIN, T** ; **BURNS, T**. Novel Graft Polymers Boost Hair Color Retention. *HAPPI*, October 2004, 92-95
- **KUMAR D** ; **KUMAR A** ; **SONDHI S** ; **SHARMA P** ; **GUPTA N**. An alkaline bacterial laccase for polymerization of natural precursors for hair dye synthesis.. *3 Biotech.*, 14 March 2018, vol. 8 (3), 182
- **JEON JR** ; **KIM EJ** ; **MURUGESAN K** ; **PARK HK** ; **KIM YM** ; **KWON JH** ; **KIM WG** ; **LEE JY** ; **CHANG YS**. Laccase-catalysed polymeric dye synthesis from plant-derived phenols for potential application in hair dyeing: Enzymatic colourations driven by homo- or hetero-polymer synthesis.. *Microb Biotechnol.*, 11 November 2009, vol. 3 (3), 324-35
- **PANWAR V** ; **DEY B** ; **SHEIKH JN** ; **DUTTA T.** Thermostable bacterial laccase for sustainable dyeing using plant phenols.. *RSC Adv.*, 21 June 2022, vol. 12 (28), 18168-18180
- **JIA W. et al.** Laccase-mediated in situ oxidation of dopamine for dyeing of human hair. *Fibers and Polymers*, 2021, vol. 22, 141-148